# EUROPÄISCHE PATENTSCHRIFT

(11) **EP 3 946 735 B1**
(45) Veröffentlichungstag und Bekanntmachung des Hinweises auf die Patenterteilung: **25.02.2026**
(21) Anmeldenummer: 20716433.6
(22) Anmeldetag: 31.03.2020
(51) Int. Cl.: B01L 3/00, A61B 10/00

(54) **VORRICHTUNG UND VERFAHREN ZUM AUFNEHMEN UND HANDHABEN EINER FLÜSSIGEN PROBE UND EINER SUBSTANZ**
DEVICE AND METHOD FOR TAKING UP AND HANDLING A LIQUID SAMPLE AND A SUBSTANCE
DISPOSITIF ET PROCÉDÉ POUR RECEVOIR ET MANIPULER UN ÉCHANTILLON LIQUIDE ET UNE SUBSTANCE

(30) Priorität: 02.04.2019 DE 102019204633
(43) Veröffentlichungstag der Anmeldung: 09.02.2022
(73) Patentinhaber: Anvajo GmbH, 01069 Dresden (DE)
(72) Erfinder: EZKERRA, Aitor, 01069 Dresden (DE); HAMMER, Daniel, 01069 Dresden (DE)
(74) Vertreter: Pfenning, Meinig & Partner mbB
(86) Internationale Anmeldenummer: PCT/EP2020/059044
(87) Internationale Veröffentlichungsnummer: WO 2020/201250

(56) Entgegenhaltungen:
- WO-A1-2016/079611
- US-A1- 2012 325 721
- US-A1- 2013 092 690
- US-A1- 2018 193 830
- US-A1- 2018 272 330

## Beschreibung

Die Erfindung betrifft eine Vorrichtung zum Aufnehmen und Handhaben einer flüssigen Probe und einer Substanz. Die Vorrichtung ist insbesondere für die Probenanalytik im medizinischen, pharmazeutischen oder biologischen Bereich geeignet.

Für die Analyse flüssiger Proben ist es häufig notwendig, eine bestimmte Menger einer flüssigen Probe verschiedenen Analyseschritten zu unterziehen. Typischerweise werden flüssige Proben hierfür mit einer Pipette oder anderen Ansaugvorrichtung, die einen Unterdruck erzeugt, aufgenommen und in ein Gefäß überführt, in dem die Analyseschritte durchgeführt werden. Die Proben können dann beispielsweise mit anderen Substanzen vermischt werden.

In vielen Fällen sind entsprechende Gerätschaften und Hilfsmittel zum Bereitstellen der Proben bzw. Substanzen, wie z. B. Pipetten, Pipettierhilfen, Schläuche, Pumpen, Wägeschiffchen oder Waagen, am Einsatz- bzw. Analyseort nicht vorhanden oder es tritt bei der Nutzung Fehlbedienung auf, sodass das Aufnehmen und Handhaben der Proben bzw. der Substanzen nicht reproduzierbar ist. Auch müssen die Gerätschaften und Hilfsmittel zum Bereitstellen der Proben und Substanzen nach dem Aufnehmen und Bereitstellen der Proben bzw. der Substanzen entsorgt oder aufwendig gereinigt werden, um Kontaminationen zu vermeiden. Eine besondere Herausforderung stellt hierbei das reproduzierbare Aufnehmen und Handhaben von Probe mit einem Volumen im Mikroliterbereich dar, da bereits geringe Abweichungen im Probenvolumen oder geringe Mengen an Verunreinigungen zu großen Fehlern in den Analyseergebnissen führen können. Aus der Druckschrift WO 2016/079611 A1 ist eine Aufnahmevorrichtung für Biopsieproben bekannt. Die Druckschrift US 2018/0272330 A1 offenbart ebenfalls eine Probenaufnahmevorichtung zur sicheren Aufbewahrung einer Probe. Aus US 2018/0193830 A1 ist ein weiterer Probenaufnahmebehälter für biologische Proben bekannt. US 2013/0092690 A1 offenbart eine Verschlusskappenanordnung zur Verwendung mit einem Behälter für biologische Proben Allen diesen Druckschriften ist, ebenso wie dem Dokument US 2012/0325721 A1 gemeinsam, dass Vorrichtungen gezeigt sind, bei denen eine Probe in einem Behältnis mit einer Substanz in Kontakt gebracht wird, indem eine Dichtmembran, die die Probe gegenüber der Substanz abdichtet bzw. abtrennt, mit einem Stanzer oder Lochdorn durchstoßen wird.

Der vorliegenden Erfindung liegt daher die Aufgabe zugrunde, die genannten Nachteile zu überwinden und eine kostengünstige und einfach zu bedienende Vorrichtung sowie ein Verfahren zum Aufnehmen und Handhaben einer flüssigen Probe und einer Substanz bereitzustellen. Insbesondere soll eine Vorrichtung als Massen- bzw. Einwegartikel bereitgestellt werden, mit der eine flüssige Probe im Mikroliterbereich reproduzierbar aufgenommen und für eine Vielzahl verschiedener Analyseschritte, u.a. das Mischen mit einer Substanz, zugängig gemacht werden kann.

Die Aufgabe wird erfindungsgemäß gelöst durch eine Vorrichtung nach Anspruch 1 und ein Verfahren nach Anspruch 11. Vorteilhafte Ausgestaltungen und Weiterbildungen sind in den abhängigen Ansprüchen beschrieben.

Eine Vorrichtung zum Aufnehmen und Handhaben einer flüssigen Probe und einer Substanz weist eine Probeneinheit mit einem Öffner und einem Probenaufnehmer auf, der ausgestaltet ist, eine flüssige Probe aufzunehmen und zu halten. Außerdem weist die Vorrichtung ein Substanzbehältnis mit einem Reservoir auf, das ausgebildet ist, eine Substanz im Reservoir aufzubewahren. Das Reservoir besitzt eine Öffnung, die von einer Dichtfolie oder Dichtplatte verschlossen ist, sodass die im Reservoir aufbewahrte Substanz gegen die Umgebung abgedichtet ist. Der Öffner der Probeneinheit ist ausgebildet, zumindest teilweise in diese Öffnung des Reservoirs eingeführt zu werden und die Dichtfolie oder Dichtplatte von der Öffnung abzuheben. Dabei ist eine in Richtung der Dichtfolie oder Dichtplatte angeordnete Fläche des Öffners, die in die Öffnung eingeführt wird und die Dichtfolie oder Dichtplatte von der Öffnung abhebt, so ausgebildet, dass die Fläche bei Kontakt mit der Dichtfolie oder Dichtplatte in einem schrägen Winkel zur Oberfläche der Dichtfolie oder Dichtplatte ausgerichtet ist und die Dichtfolie oder Dichtplatte beim Einführen in die Öffnung sukzessive von der Öffnung abhebt, sodass ein Auslass in der Dichtfolie oder Dichtplatte gebildet wird, durch den die Substanz aus dem Reservoir austritt und mit der Probe des Probenaufnehmers mischbar ist.

Aufgrund des integrierten Probenaufnehmers sind neben der Vorrichtung keine separaten Gerätschaften oder Hilfsmittel für die Aufnahme der flüssigen Probe notwendig. Dies hat den Vorteil, dass die Vorrichtung autark zum Aufnehmen von Proben am Einsatzort genutzt werden kann. Außerdem ist eine Kontamination der Probe durch separate Gerätschaften oder Hilfsmittel zum Aufnehmen der Probe ausgeschlossen und das Reinigen dieser Gerätschaften oder Hilfsmittel kann entfallen.

Durch die Bereitstellung der Substanz in einem abgedichteten Reservoir lassen sich Kontaminationen der Substanz, aber auch Kontaminationen der Umgebung vermeiden. Insbesondere gefährliche Substanzen, wie z. B. biogefährliche oder krebserregende Stoffe, können mit der Vorrichtung sicher, ohne unkontrollierten Kontakt zur Umgebung transportiert und gehandhabt werden. Das Reservoir kann außerdem an unterschiedlichen Orten befüllt und entleert werden. Es kann beispielsweise in einer sterilen Umgebung mittels Hochpräzisionsvorrichtungen befüllt und verschlossen werden, um dann in der Vorrichtung an einem anderen Ort verwendet zu werden. Dadurch kann einerseits der Messfehler beim Befüllen des Substanzbehältnisses verringert werden, andererseits ist die Vorrichtung am Einsatzort autark von Gerätschaften oder Hilfsmittel zur Befüllung nutzbar.

Das Reservoir kann mit einer festen, flüssigen und bzw. oder gasförmigen Substanz, insbesondere einem Fluid oder mehreren Fluiden, wie z. B. einer fließfähigen Flüssigkeit, einem fließ- oder rieselfähigen Feststoff, wie einem Pulver, Granulat oder Agglomerat, oder Mischungen aus diesen befüllbar bzw. befüllt sein. Es kann aber auch einen zähflüssigen Stoff, wie z. B. ein Gel oder eine Paste, oder einen festen Stoff aufnehmen, der mittels der Probe oder einer anderen Flüssigkeit aus dem Reservoir herausgespült werden kann.

Der schräge Winkel zwischen der Dichtfolie oder Dichtplatte und der Fläche am Öffner, die in die Öffnung des Reservoirs eingeführt wird und die Dichtfolie oder Dichtplatte von der Öffnung abhebt, verhindert ein unkontrolliertes Einreißen der Dichtfolie oder Dichtplatte. Statt unkontrolliert einzureißen und abschnittsweise zur Seite gedrängt zu werden, wird die Dichtfolie oder Dichtplatte als zusammenhängende Fläche sukzessive von der Öffnung abgehoben, sodass der entstehende Auslass vollständig für das Freisetzen der Substanz aus dem Reservoir genutzt werden kann. Hierfür genügt typischerweise bereits eine einzige lineare Bewegung des Öffners, diese kann insbesondere in Normalenrichtung der Öffnungsfläche der Öffnung erfolgen, die hierfür in der Regel parallel zur Normalenrichtung einer Endfläche des Probenaufnehmers geführt wird. Bevorzugt bilden die Fläche des Öffners, die in die Öffnung des Reservoirs eingeführt wird und die Dichtfolie oder Dichtplatte von der Öffnung abhebt, bei Kontakt mit der Dichtfolie oder Dichtplatte einen Winkel zwischen 5° und 45°, bevorzugt 5° und 30°, besonders bevorzugt 5° und 20° zur Oberfläche der Dichtfolie oder Dichtplatte aus.

Es kann vorgesehen sein, dass die Fläche des Öffners, die in die Öffnung des Reservoirs eingeführt wird und die Dichtfolie oder Dichtplatte von der Öffnung abhebt, ausgebildet ist, die Dichtfolie oder Dichtplatte sukzessive vom Rand der Öffnung beginnend abzuheben. Dies hat den Vorteil, dass die vom Öffner abgehobene Dichtfolie oder Dichtplatte beim Einführen des Öffners in die Öffnung an die Seitenwand der Öffnung bzw. Innenwand des Reservoirs bewegt wird und dadurch beim Freisetzen der Substanz aus dem Reservoir nur einen verschwindend geringen Strömungswiderstand darstellt. Das Reservoir kann somit schnell und vollständig entleert werden. Hierbei ist insbesondere die Möglichkeit günstig, dass die Dichtfolie oder Dichtplatte beim Einführen des Öffners in die Öffnung sukzessive von der gesamten Öffnung als zusammenhängende Fläche abgehoben wird und an die Seitenwand bzw. Innenwand des Reservoirs bewegt bzw. umgeklappt und dort von dem Öffner fixiert werden kann. Dadurch kann der Strömungswiderstand der Dichtfolie oder Dichtplatte beim Freisetzen der Substanz aus dem Reservoir verringert werden.

Vorteilhaft ist es, wenn der Öffner eine Spitze, einen Vorstecher, einen Lochdorn bzw. eine Schneidkante aufweist, womit die Dichtfolie oder Dichtplatte bei Kontakt mit der Dichtfolie oder Dichtplatte durchtrennt. Mit der Spitze, dem Vorstecher, dem Lochdorn bzw. der Schneidkante kann der Druck, der aufgebracht werden muss, um die Dichtfolie oder Dichtplatte zum Abheben zu durchtrennen, verringert und somit das Freisetzen der Substanz erleichtert werden. Darüberhinaus kann ein unkontrolliertes Einreißen aber auch ein ungewolltes Einwölben und Zusammenrollen der Dichtfolie oder Dichtplatte verhindert werden.

Bevorzugt ist wenigstens eine Spitze, ein Vorstecher oder ein Lochdorn an der Stelle des Öffners angeordnet, die beim Einführen des Öffners in die Öffnung die Dichtfolie oder Dichtplatte zuerst berührt. Die Schneidkante ist bevorzugt am Rand der Fläche des Öffners, die in die Öffnung eingeführt wird und die Dichtfolie oder Dichtplatte von der Öffnung abhebt, ausgebildet. Die Schneidkante kann insbesondere mit einer einseitigen oder beidseitigen, flachen, balligen, hohlförmigen oder konkaven Schneidgeometrie ausgebildet sein. Außerdem kann es vorgesehen sein, dass die Schneidkante Schneidzähne oder eine Mikrofase aufweist, die einen besonders sauberen Schnitt ermöglicht.

Der Öffner der Probeneinheit ist vorzugsweise komplementär geformt ausgebildet zu der Öffnung des Reservoirs, in die er zum Abheben der Dichtfolie oder Dichtplatte eingeführt wird, d. h. der Öffner ist insbesondere passend bzw. passgenau geformt zur Öffnung ausgebildet. Dadurch kann die Dichtfolie oder Dichtplatte im Wesentlichen über den gesamten Querschnitt der Öffnung abgehoben und für das Freisetzen der Substanz aus dem Reservoir geöffnet werden. Die Dichtfolie oder Dichtplatte kann außerdem beim sukzessiven Einführen des Öffners und Abheben der Dichtfolie oder Dichtplatte an die Seitenwand der Öffnung und bzw. oder die Innenwand des Reservoirs bewegt werden und dort durch den Öffner fixiert werden, sodass sie beim Austreten der Substanz nicht von dieser mit in Richtung des Auslasses bewegt wird und diesen nicht versperrt. Der Öffner und insbesondere die in Richtung der Dichtfolie oder Dichtplatte angeordnete Fläche des Öffners kann also eine Außenkontur bzw. Außenkante aufweisen, die derart geformt ist, dass bei Einführen des Öffners in die Öffnung ein Spalt zwischen der Außenkontur und bzw. oder Außenkante und einer Seitenwand der Öffnung und bzw. oder der Innenwand des Reservoirs gebildet wird, in dem die Dichtfolie oder Dichtplatte fixiert ist. Unter dem Begriff fixiert kann in dieser Anmeldung insbesondere verstanden werden, dass die Bewegung der Dichtfolie oder Dichtplatte durch eine Außenkontur des Öffners und die Seitenwand der Öffnung und bzw. oder die Innenwand des Reservoirs derart eingeschränkt ist, dass die Dichtfolie oder Dichtplatte beim Austreten der Substanz nicht von dieser mit in Richtung der Öffnung bewegt wird. Die Dichtfolie oder Dichtplatte kann dabei insbesondere an der Seitenwand der Öffnung und bzw. oder der Innenwand des Reservoirs flach anliegen. Der Öffner und bzw. oder die in Richtung der Dichtfolie oder Dichtplatte angeordnete Fläche des Öffners kann beispielsweise derart ausgebildet sein, dass die Außenkontur bzw. Außenkante bei Einführen des Öffners in die Öffnung einen Spalt bildet, dessen Spaltmaß das 1,5-fache bis 20-fache der Dicke der Dichtfolie oder Dichtplatte, bevorzugt das 5-fache bis 10-fache der Dicke der Dichtfolie oder Dichtplatte, beträgt.

Das Reservoir ist bevorzugt als einseitig offene Kavität ausgebildet, damit durch das Öffnen der Dichtfolie oder Dichtplatte eine möglichst große Querschnittsfläche für das Freisetzen der Substanz aus dem Reservoir entstehen kann. Dies hat den Vorteil, dass selbst zähflüssige oder feste Substanzen aus dem Reservoir durch ein Herausspülen mittels der Probe oder einer anderen Flüssigkeit schnell und unkompliziert freigesetzt werden können. Besonders bevorzugt ist die Öffnung im Reservoir an der Unterseite des Reservoirs bzw. des Substanzbehältnisses ausgebildet, sodass ein ungehindertes Austreten der Substanz mittels der Schwerkraft möglich ist.

Es ist vorteilhaft, wenn der Öffner, der in die Öffnung des Reservoirs eingeführt wird, mindestens eine Durchführung und bzw. oder mindestens eine Austrittsöffnung aufweist, durch die die Substanz aus dem Reservoir beim Abheben der Dichtfolie oder Dichtplatte austreten kann. Der Öffner ist als Ring bzw. ringförmig ausgebildet, der über mehrere Stege mit dem Probenaufnehmer verbunden ist.

Dadurch kann vermieden werden, dass der Öffner die Substanz am Austreten durch den Auslass hindert. Die Substanz kann bereits während des Durchdringens der Dichtfolie oder Dichtplatte aus dem Reservoir austreten.

Unter dem Begriff "Ring" soll hierbei insbesondere ein gleichmäßig runder, in sich geschlossener Gegenstand beliebiger Breite und bzw. oder Länge verstanden werden. Mit Austrittsöffnungen, die in verschiedene Raumrichtungen ausgerichtet sind, kann außerdem eine homogenere Verteilung der austretenden Substanz erreicht werden, was insbesondere das Mischen der Substanz mit einer Probe im Bereich des Öffners begünstigt. Dem Begriff "Steg" soll im Rahmen dieser Anmeldung insbesondere ein Verbindungsteil verstanden werden, dass jedoch einen Raum zwischen zwei durch diesen Steg zu verbindenden Elementen nicht komplett füllt und bzw. oder überdeckt.

Der Probenaufnehmer kann als Kapillarröhrchen ausgestaltet sein, das ausgebildet ist, eine definierte Probenmenge mittels Kapillarkräften in sich aufzunehmen. Kapillarröhrchen sind kostengünstige und ausfallsichere Probenaufnehmer, da sie weder elektronische noch mechanisch bewegte Komponenten benötigen. Aufgrund des geringen Öffnungsquerschnitts der Kapillarröhrchen können kaum Feststoffe in die Kapillarröhrchen eindringen, sodass die Probe weitestgehend ohne Kontaminierung aufgenommen werden kann. Typische Durchmesser und Fassungsvolumina der Kapillarröhrchen betragen zwischen 0,02 mm und 2 mm und 1 µl bis 100 µl.

Vorzugsweise ist der Probenaufnehmer gegenüberliegend zum Öffner an der Probeneinheit angeordnet. Für flüssige Substanzen kann der Probenaufnehmer beispielsweise mittig zur Fläche des Öffners, die in die Öffnung des Reservoirs eingeführt wird und die Dichtfolie oder Dichtplatte von der Öffnung abhebt, angeordnet sein, sodass er beim Austreten der Substanz aus dem Reservoir von dieser um- bzw. durchströmt werden kann und eine Durchmischen der Substanz mit der Probe aus dem Probenaufnehmer erleichtert wird. Für fließ- bzw. rieselfähige feste Substanzen kann der Probenaufnehmer auch seitlich versetzt zur Mitte der Fläche, die in die Öffnung des Reservoirs eingeführt wird und die Dichtfolie oder Dichtplatte von der Öffnung abhebt, angeordnet sein, damit die Substanz den Probenaufnehmer beim Austreten aus dem Reservoir nicht verstopft.

Der Probenaufnehmer und der Öffner können als zusammenhängende Einheit ausgebildet sein. Der Probenaufnehmer kann beispielsweise über Stege am Öffner verbunden sein, wobei die Stege einen möglichst schmalen Querschnitt aufweisen, vorzugsweise schmaler als 2 mm, damit sie das Austreten der Substanz aus dem Reservoir nicht behindern. Die Stege können eine Außenkontur aufweisen, die komplementär, und bevorzugt passgenau, zu einer Seitenwand der Öffnung und bzw. oder Innenwand des Reservoirs geformt ist. Besonders bevorzugt kann mindestens ein Steg am Öffner in einem Bereich angeordnet sein, in dem die Fläche des Öffners bei Kontakt mit der Dichtfolie oder Dichtplatte einen maximalen Abstand zur Öffnung aufweist, sodass die Dichtfolie oder Dichtplatte beim Einführen des Öffners in die Öffnung von diesem Steg an die Seitenwand der Öffnung und bzw. oder die Innenwand des Reservoirs bewegt und fixiert wird.

Alternativ kann die Probeneinheit auch modular aufgebaut sein, sodass sich beispielsweise unterschiedliche Probenaufnehmer mittels einer form- bzw. kraft- oder reibschlüssigen Durchsteckverbindung an der Probeneinheit befestigen lassen. In einer Ausführungsform kann auch vorgesehen sein, dass die Probeneinheit mehrere Probenaufnehmer aufweist, sodass verschiedene Flüssigkeiten bzw. größere Mengen an Flüssigkeiten aufgenommen werden können.

Für eine unkomplizierte Handhabung der Vorrichtung kann es außerdem vorgesehen sein, dass das Substanzbehältnis an der Probeneinheit befestigbar ist und das Substanzbehältnis bzw. das Probenbehältnis ausgebildet ist, den Öffner erst mittels einer Druck- bzw. Drehbewegung am Substanzbehältnis oder an der Probeneinheit in die Öffnung einzuführen und die Dichtfolie oder Dichtplatte von der Öffnung abzuheben. Die Vorrichtung lässt sich somit als Einheit aus Substanzbehältnis und Probeneinheit transportieren bzw. handhaben und die Substanz erst bei Bedarf freisetzen.

Es kann insbesondere vorgesehen sein, dass die Vorrichtung eine Befestigungs- und Auslösevorrichtung aufweist, mit der das Substanzbehältnis so an der Probeneinheit befestigbar ist, dass die relative Bewegung des Substanzbehältnisses zum Probenbehältnis für das Einführen des Öffners in die Öffnung des Reservoirs und das Abheben der Dichtfolie oder Dichtplatte blockiert ist und erst nach Tätigen der Auslösevorrichtung freigeben ist. Die Befestigungs- und Auslösevorrichtung kann beispielsweise als Einrastvorrichtung mit einem lösbaren Einklick- oder Schnapp-Mechanismus ausgebildet sein oder als Schraubverbindung mit einem Verriegelungsmechanismus wie einem Stift, Zapfen, Splint oder Hebel, der die relative Bewegung des Substanzbehältnisses zum Probenbehältnis für das Einführen des Öffners in die Öffnung und das Abheben der Dichtfolie oder Dichtplatte erst nach dessen Entfernen oder Betätigen freigibt. Die Auslösevorrichtung ist vorzugsweise rein manuell bedienbar, wobei für bestimmte Anwendungen auch eine mechanische Kindersicherung der Auslösevorrichtung vorgesehen sein kann.

Bevorzugt weist die Vorrichtung, insbesondere die Probeneinheit, auch eine Verbindungsstelle auf, an der ein Behältnis befestigbar ist, das den Probenaufnehmer und die Öffnung des Reservoirs umschließt. In einem solchen Behältnis können z. B. die Substanz und die Probe aufgenommen und miteinander gemischt werden, das Behältnis kann aber auch dazu verwendet werden, die Probe vor dem Austrocken zu schützen oder eine Flüssigkeit zum Ausspülen zähflüssiger oder fester Substanzen aus dem Reservoir bereitzustellen.

Besonders bevorzugt ist die Verbindungsstelle ausgebildet, die Vorrichtung lösbar mit Standardlaborgefäßen, z. B. Küvetten, Gefäßen mit Normschliff, Zentrifugierröhrchen oder Eppendorfgefäßen, oder mit Laborgeräten, die eine zu Standardlaborgefäßen kompatible Verbindungsstelle aufweisen, zu verbinden. Die Verbindungsstelle kann beispielsweise für Schraubverbindungen, Einrast- oder Schnappverbindungen ausgestaltet sein. Besonders vorteilhaft ist die Verbindungsstelle für reibschlüssige Einschub- bzw. Aufsteckverbindungen ausgestaltet, sodass die Vorrichtung schnell und unkompliziert mit einem Behältnis befestigt werden kann.

Die Probeneinheit kann beispielsweise einen Mantel, eine Hülse oder einen Ring aufweisen, der um den Öffner angeordnet ist und als Verbindungsstelle ausgebildet ist. Eine Außenfläche des Mantels, der Hülse oder des Rings kann hierfür beispielsweise als Dichtfläche für eine reibschlüssige Befestigung eines Behältnisses ausgestaltet sein, das auf den Mantel, die Hülse oder den Ring aufgeschoben wird. Der Mantel, die Hülse oder der Ring können außerdem einen Berührungsschutz bilden, der den Öffner, wenn die Probeneinheit auf einer Fläche abgelegt wird oder gehandhabt wird, vor ungewollten Berührungen schützt. Ferner kann es vorgesehen sein, dass der Mantel, die Hülse oder der Ring eine Außenfläche aufweisen, die als Haltefläche für das Handhaben der Probeneinheit, insbesondere für das Halten der Probeneinheit während der Aufnahme der flüssigen Probe, ausgestaltet ist.

Der Mantel, die Hülse oder der Ring können beispielsweise mittels schmaler Stege am Öffner befestigt sein und eine Einheit mit dem Öffner bilden. In einer Ausführungsform kann der Mantel, die Hülse oder der Ring eine Innenfläche aufweisen, die komplementär geformt zu einer Außenfläche des Substanzbehältnis oder Reservoirs ausgestaltet ist, sodass die Probeneinheit und das Reservoir an dieser Fläche beim Einführen des Öffners in die Öffnung des Reservoirs und Abheben der Dichtfolie oder Dichtplatte von der Öffnung formschlüssig gegen die Umgebung abgedichtet werden können und ein unkontrolliertes Austreten der Substanz aus der Vorrichtung vermieden werden kann. Anstelle oder zusätzlich zu dieser Abdichtung können am Substanzbehältnis bzw. der Probeneinheit weitere Dichtflächen vorgesehen sein, an denen das Substanzbehältnis und das Probenbehältnis beim Einführen des Öffners in die Öffnung des Reservoirs und Abheben der Dichtfolie oder Dichtplatte von der Öffnung gegebenenfalls mittels eines zusätzlichen Dichtmaterials gegen die Umgebung abgedichtet werden können.

In einer Ausführungsform kann es ferner vorgesehen sein, dass das Substanzbehältnis mehrere Reservoirs und die Probeneinheit mehrere Öffner aufweist. Die Reservoirs und Öffner können dabei derart im Substanzbehältnis bzw. an der Probeneinheit angeordnet sein, dass je ein Öffner in eine Öffnung eines Reservoirs einführbar ist und die Dichtfolie oder Dichtplatte des Reservoirs öffnet. Die Probeneinheit kann entsprechend eine Verbindungsstelle aufweisen, an der ein Behältnis befestigbar ist, das den Probenaufnehmer und die Öffnungen der Reservoirs umschließt. Somit ist es möglich, verschiedene Substanzen bereitzustellen, die erst nach Öffnen der Dichtfolie oder Dichtplatte des jeweiligen Reservoirs miteinander mischbar sind bzw. vermischt werden.

Insbesondere kann es vorgesehen sein, dass die Öffner unterschiedliche Längen aufweisen oder an der Probeneinheit in unterschiedlicheren Ebenen angeordnet sind, sodass sie beim Einführen in die Öffnungen der Reservoirs unterschiedliche Abstände zur Ebene der Dichtfolien oder Dichtplatten der Reservoirs aufweisen. Die Dichtfolien oder Dichtplatten der Reservoirs können somit beim Einführen der Öffner in die Öffnungen der Reservoirs nacheinander und unabhängig voneinander geöffnet werden, wobei das Reservoir mit dem kleinsten Abstand zwischen dem Öffner und der Dichtfolie oder Dichtplatte als erstes geöffnet wird.

Alternativ können die Öffner gleich lang ausgestaltet sein und in einer Ebene an der Probeneinheit angeordnet sein und die Reservoirs bzw. die Dichtfolien oder Dichtplatten der Reservoirs in unterschiedlichen Ebenen zueinander im Substanzbehältnis angeordnet sein, sodass die Dichtfolien oder Dichtplatten beim Einführen der Öffner in die Öffnungen der Reservoirs nacheinander und unabhängig voneinander geöffnet werden können, wobei das Reservoir mit dem kleinsten Abstand zwischen dem Öffner und der Dichtfolie oder Dichtplatte als erstes geöffnet wird. Die Vorrichtung kann entsprechend mehrere Verriegelungs- und Auslöseeinheiten aufweisen, die nacheinander betätigt werden können, sodass die Öffner nacheinander in die Öffnungen der Reservoirs eingeführt werden können und die Substanzen nacheinander freigesetzt werden können. Dadurch können bestimmte Reihenfolgen, in denen die Substanzen freigesetzt werden sollen, festgelegt werden und so z. B. Verwechslungen oder Fehler in Analyseabfolgen vermieden werden. Die Vorrichtung eignet sich außerdem besonders für das zeitgesteuerte Freisetzen von Substanzen. Die Substanzen können beispielsweise manuell oder automatisch in bestimmten Zeitabständen freigesetzt werden.

Die Probeneinheit, das Substanzbehältnis und die Dichtfolie bzw. Dichtplatte zur Abdichtung des Reservoir besteht typischerweise aus Werkstoffen, die eine Langzeitaufbewahrung der Substanz bzw. der Probe ermöglichen, d. h. die chemisch bzw. thermisch beständig sind und die Substanz bzw. Probe gegebenenfalls gegen Umwelteinflüsse, wie UV-Strahlung, Feuchtigkeit oder Sauerstoff schützen. Die Probeneinheit bzw. das Substanzbehältnis können aus Kunststoffen hergestellt sein, z. B. aus themoplastischen Kunststoffen wie Polypropylen, Polyethylen, Polyethylenterephthalat, Polystyrol, Cycloolefin-Polymeren bzw. Copolymeren, Acrylnitrilbutadienstyrol, Polyetheretherketon, Polycarbonat, Polyamiden, insbesondere Nylon, Polyoxymethylen oder Polyacryl, oder duroplastischen Kunststoffen, wie Epoxid-, Polyurethan- oder Phenolharzen. Die Probeneinheit und das Substanzbehältnis sind vorzugsweise durch Spitzgussverfahren hergestellt, können aber auch durch additive Fertigungstechniken, wie 3D-Druck oder Sintern, Lamination, Extrusion, Kleben, Fräsen, Zerspanen oder Ablation hergestellt sein.

Die Dichtfolie oder Dichtplatte zur Abdichtung des Reservoir besteht typischerweise aus einem wasser- bzw. luftundurchlässigen Werkstoff, insbesondere einem Metall, beispielsweise Aluminium, oder einem nicht-elastischen Kunststoff, d. h. einem Kunststoff mit einem Elastizitätsmodul größer als 0,1 GPa bei 20 °C . Die Dicke der Dichtfolie oder Dichtplatte beträgt typischerweise zwischen 10 µm und 200 µm, vorzugsweise zwischen 20 µm und 100 µm. Die Dichtfolie oder Dichtplatte kann zum Abdichten der Öffnung im Reservoir beispielsweise auf die Ränder der Öffnung aufgeklebt, laminiert oder geschweißt sein.

Bei einem Verfahren zum Aufnehmen und Handhaben einer flüssigen Probe und einer Substanz wird eine flüssige Probe mit einem Probenaufnehmer, der an einer Probeneinheit angeordnet ist, aufgenommen und ein Substanzbehältnis bereitgestellt, in dem in einem Reservoir eine Substanz aufbewahrt ist. Anschließend wird ein Öffner, der als durchbrochener Hohlkörper oder als Ring ausgebildet und an der Probeneinheit angeordnet ist, in eine Öffnung des Reservoirs eingeführt, die von einer Dichtfolie oder Dichtplatte verschlossen ist. Dabei ist eine Fläche am Öffner, die in Richtung der Dichtfolie oder Dichtplatte angeordnete ist, bei Kontakt mit der Dichtfolie oder Dichtplatte in einem schrägen Winkel zur Oberfläche der Dichtfolie oder Dichtplatte ausgerichtet und hebt die Dichtfolie oder Dichtplatte sukzessive von der Öffnung des Reservoirs ab, sodass eine Auslass gebildet wird, durch den die Substanz aus dem Reservoir austreten kann und mit der Probe des Probenaufnehmers mischbar ist.

Das beschriebene Verfahren kann insbesondere mit der beschriebenen Vorrichtung durchgeführt werden, das heißt, die beschriebene Vorrichtung ist zum Durchführen des beschriebenen Verfahrens eingerichtet.

Ausführungsbeispiele der Erfindung sind in den Zeichnungen dargestellt und werden nachfolgend anhand der Figuren 1 bis 10 erläutert.

Es zeigen:
- Fig. 1: in einer schematischen, seitlichen Ansicht ein Beispiel einer erfindungsgemäßen Vorrichtung,
- Fig. 2: in einer Figur 1 entsprechenden Ansicht ein Beispiel einer erfindungsgemäßen Vorrichtung nach einer möglichen Verwendung,
- Fig. 3: in einer schematischen, seitlichen Teilschnittansicht ein weiteres Beispiel einer erfindungsgemäßen Vorrichtung,
- Fig. 4: in einer Figur dem unteren Teil von Figur 3 entsprechenden Ansicht ein Beispiel einer erfindungsgemäßen Vorrichtung mit einer offenen Schnappbefestigung,
- Fig. 5: in einer schematischen perspektivischen Ansicht ein Beispiel einer erfindungsgemäßen Vorrichtung mit einer geschlossenen Schnappbefestigung vor dem Öffnen der Dichtfolie oder Dichtplatte,
- Fig. 6: in einer schematischen, seitlichen Ansicht ein Beispiel einer erfindungsgemäßen Vorrichtung mit einer gelösten Schnappbefestigung nach dem Öffnen der Dichtfolie bzw. Dichtplatte,
- Fig. 7: eine schematische perspektivischen Ansicht ein Beispiel eines als einseitig abgeschrägten Hohlzylinder ausgestalteten Öffners,
- Fig. 8: eine Figur 7 entsprechende Ansicht des in Figur 7 gezeigten Öffners ohne Ummantelung,
- Fig. 9: eine Schnittansicht des in Figur 7 dargestellten Ausführungsbeispiels und
- Fig. 10: eine gegenüber der Darstellung in Figur 9 um 90° gedrehte schematische Schnittansicht.

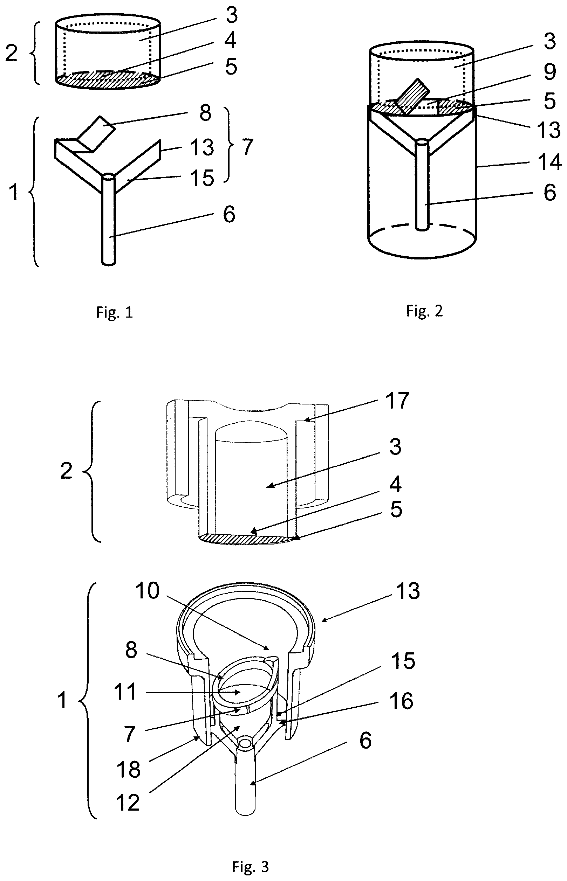

In Figur 1 und 2 ist in einer schematischen, seitlichen Ansicht ein Ausführungsbeispiel einer Vorrichtung zum Aufnehmen und Handhaben einer flüssigen Probe dargestellt. Figur 1 zeigt die Vorrichtung im zerlegten Zustand vor ihrer Verwendung und Figur 2 zeigt die Vorrichtung in einem zusammengefügten Zustand nach einer möglichen Verwendung.

Die Vorrichtung ist mit einer Probeneinheit 1 und einem Substanzbehältnis 2 ausgestattet. Das Substanzbehältnis 2 weist ein Reservoir 3 auf, das ausgebildet ist, eine Substanz im Reservoir 3 aufzubewahren. Dieses Reservoir 3 ist mit einer Öffnung 4 versehen, die von einer Dichtfolie oder Dichtplatte 5 verschlossen ist, sodass die im Reservoir 3 aufbewahrte Substanz gegen die Umgebung abgedichtet ist und im Reservoir 4 verbleibt. Das Reservoir 3 ist somit eine durch die Dichtplatte 5 geschlossene Kavität. Die Probeneinheit 1 verfügt über einen Öffner 7 und einen Probenaufnehmer 6, der ausgestaltet ist, eine flüssige Probe aufzunehmen und zu halten. Der Öffner 7 der Probeneinheit 1 ist ausgebildet, zumindest teilweise bzw. vollständig in die Öffnung 4 des Reservoirs 3 eingeführt zu werden und die Dichtfolie oder Dichtplatte 5 von der Öffnung 4 abzuheben. Dabei ist eine in Richtung der Dichtfolie oder Dichtplatte 5 angeordnete Fläche 8 des Öffners 7, die in die Öffnung 4 des Reservoirs 3 eingeführt wird und die Dichtfolie oder Dichtplatte 5 von der Öffnung 4 abhebt, so ausgebildet, dass die Fläche 8 bei Kontakt mit der Dichtfolie oder Dichtplatte 5 in einem schrägen Winkel, d.h. insbesondere einem von 0° und 90° verschiedenen Winkel, zur Oberfläche der Dichtfolie oder Dichtplatte 5 ausgerichtet ist und die Dichtfolie oder Dichtplatte 5 beim Einführen in die Öffnung 4 sukzessive von der Öffnung 4 abhebt, sodass ein sich sukzessive, also mit zunehmendem Eindringen des Öffners 7, vergrößernder Auslass 9 in der Dichtfolie oder Dichtplatte 5 gebildet wird, durch den die Substanz aus dem Reservoir 3 austritt und mit der Probe des Probenaufnehmers 6 mischbar ist.

Durch den schrägen Winkel zwischen der Fläche 8 des Öffners 7 und der Dichtfolie bzw. Dichtplatte 5 kann die Dichtfolie bzw. Dichtplatte 5 ohne unkontrolliert einzureißen vom Rand der Fläche 8 sukzessive, d. h. nach und nach, durchtrennt und als zusammenhängende Fläche von der Öffnung 4 des Reservoirs 3 abgehoben werden. Im dargestellten Beispiel beträgt der Winkel bei Kontakt des Öffners 7 mit der Dichtfolie bzw. Dichtplatte 5 45°, es können jedoch auch kleinere Winkel als 45°, bevorzugt zwischen 5° und 30°, besonders bevorzugt zwischen 5° und 20° vorgesehen sein.

Das Reservoir 3 des Substanzbehältnisses 2 kann typischerweise ein Volumen von 0,5 ml bis 5 ml fassen. Das Reservoir 3 kann, wie in den Figuren 1 und 2 dargestellt, als einseitig offene Kavität ausgebildet sein, wobei sich die Öffnung 4 des Reservoirs 3 an der Unterseite des Substanzbehältnisses 2 befinden kann, sodass die Substanz beim Öffnen der Dichtfolie oder Dichtplatte 5 mittels der Schwerkraft ungehindert aus dem Reservoir 3 austreten kann. Der Öffner 7 wird im gezeigten Beispiel dementsprechend von unten in die Öffnung 4 des Reservoirs 3 eingeführt, bzw. das Substanzbehältnis 2 von oben auf die Probeneinheit 1 aufgesetzt, um einen Kontakt zwischen dem Öffner 7 und der Dichtfolie bzw. Dichtplatte 5 herzustellen und die Dichtfolie der Dichtplatte 5 sukzessive von der Öffnung 4 des Reservoirs 3 abzuheben.

Die Probeneinheit 1 und das Substanzbehältnis 2 bestehen bevorzugt aus chemisch inerten Kunststoffen, im dargestellten Beispiel der Figur 1 und 2 aus Polypropylen. Das Substanzbehältnis 2 oder die Dichtfolie bzw. Dichtplatte 5 bestehen besonders bevorzugt aus thermoplastischen Kunststoffen, sodass beim Aufbringen der Dichtfolie oder Dichtplatte 5 lediglich die Dichtfolie oder Dichtplatte 5 bzw. das Substanzbehältnis 2 um die Öffnung 4 des Reservoirs 3 erwärmt werden und kein zusätzlicher Klebstoffe notwendig ist. Für die Bereitstellung von temperaturempfindlichen Substanzen oder Verwendung von Substanzbehältnissen 2 aus beispielsweise duroplastischen Kunststoffen kann die Dichtfolie bzw. Dichtplatte 5 aber auch mittels eines chemisch internen Klebstoffs auf das Substanzbehältnis 2 aufgeklebt bzw. auflaminiert sein.

Die Öffnung 4 des Reservoirs 3 ist im dargestellten Beispiel der Figuren 1 und 2 mit einer 20 µm dicken Dichtfolie 5 aus Aluminium verschlossen, die auf den Rand der Öffnung 4 unter Pressen aufgeschweißt ist. Anstelle einer Aluminiumfolie können auch andere nicht-elastische Werkstoffe, insbesondere Kunststoffe für die Dichtfolie bzw. Dichtplatte 5 verwendet werden, insofern sie chemisch inert und undurchlässig für die Substanz und Luft bzw. Wasser sind. Die Dicke der Dichtfolie bzw. Dichtplatte 5 ist dabei so gewählt, dass die Dichtfolie bzw. Dichtplatte 5 mit dem Öffner 7 bereits durch einen manuell ausgeübten Druck auf die Probeneinheit 1 bzw. das Substanzbehältnis 2 durchtrennt und von der Öffnung 4 im Reservoir 3 abgehoben werden kann.

Im dargestellten Beispiel der Figuren 1 und 2 ist der Probenaufnehmer 6 fest über schmale Stege 15 mit dem Öffner 7 verbunden und bildet eine Einheit mit dem Öffner 7, ist also einteilig bzw. in stoffschlüssiger Verbindung mit diesem ausgebildet. Es kann jedoch auch vorgesehen sein, dass der Probenaufnehmer 6 lösbar, z. B. über eine Steckverbindung, mit dem Öffner 7 verbunden ist.

Der Probenaufnehmer 6 kann an der Probeneinheit 1, wie in den Figuren 1 und 2 dargestellt, derart ausgebildet sein, dass der Probenaufnehmer 1 dem durch den Öffner 7 gebildeten Auslass 9 gegenüberliegend angeordnet ist, also insbesondere mittig an der Probeneinheit 1 angeordnet ist. In dieser Anordnung kann die Substanz beim Austreten aus dem Reservoir 3 die Probe aus dem Probenaufnehmer 1 spülen. Dies ist besonders für flüssige oder gelöste Substanzen von Vorteil, die in einem nachfolgenden Analyseschritt mit der Probe vermischt werden. Es kann jedoch auch vorgesehen sein, dass der Probenaufnehmer 6 in Draufsicht nicht mittig, sondern seitlich versetzt an der Probeneinheit 1 angeordnet ist, um z. B. ein Verstopfen des Probenaufnehmers 6 oder einen zu hohen Strömungswiderstand in Auslassrichtung der Substanz zu verhindern.

Wie in Figur 1 und 2 dargestellt, kann der Probenaufnehmer 6 beispielsweise als Kapillarröhrchen ausgebildet sein, das bei Kontakt mit einer flüssigen Probe ein definiertes Volumen dieser Flüssigkeit mittels Kapillarkräften aufnimmt und hält. Ein solcher passiver Probenaufnehmer 6 ohne elektronische oder mechanisch bewegte Elemente ist ausfallsicher, kostengünstig und einfach zu bedienen. Typische Durchmesser und Fassungsvolumina der Kapillarröhrchen betragen zwischen 0,02 mm und 2 mm bzw. 1 µl bis 100 µl. Die Enden der Kapillarröhrchen können für eine sehr präzise Volumenabmessung der Probe als ebene, senkrecht zur Längsachse des Kapillarröhrchens verlaufenden Flächen ausgebildet sein, wobei ein Ende des Kapillarröhrchens, wie in den Figuren 1 und 2 gezeigt, für das Aufnehmen der flüssigen Probe aus der Probeneinheit 1 hervorragt.

Im Beispiel der Figuren 1 und 2 verfügt die Vorrichtung auch über eine Verbindungsstelle 13 für ein Behältnis 14. Die Verbindungsstelle 13 ist an einander gegenüberliegenden Außenfläche des Öffners 7 ausgebildet, sodass daran ein Behältnis 14 befestigt werden kann, das die Öffnung 4 des Reservoirs 3 und den Probenaufnehmer 6 umschließt. Das Behältnis 14 kann, wie in Figur 2 dargestellt, auf die Probeneinheit 1 aufgesteckt werden und die Probe im Probenaufnehmer 6 reibschlüssig gegen die Umgebung abdichten. Anstelle einer Steckverbindung kann beispielsweise auch eine Verbindungsstelle 13 für eine Schraub-, Einrast- oder Schnappverbindung vorgesehen sein. Bevorzugt ist die Verbindungsstelle 13 so ausgestaltet, dass die Vorrichtung mit Standardlaborgefäßen, wie z. B. Küvetten, Gefäßen mit Normschliff, Zentrifugierröhrchen oder Eppendorfgefäßen, verbunden werden kann, insbesondere auf eine Öffnung dieser Gefäße auf- oder eingesteckt werden kann, oder mit Laborgeräten, die eine Verbindungsstelle für Standardlaborgefäße aufweisen, verbunden werden kann.

Figur 3 zeigt in einer schematischen, seitlichen Teilschnittansicht ein weiteres Beispiel einer Vorrichtung. Wiederkehrende Merkmale sind in dieser Figur, wie auch in den folgenden Figuren, mit identischen Bezugszeichen versehen. In der Vorrichtung der Figur 3 ist die Fläche 8 des Öffners 7, die in die Öffnung 4 des Reservoirs 3 eingeführt wird und die Dichtfolie oder Dichtplatte 5 von der Öffnung 4 abhebt, so ausgebildet, dass die Fläche 8 die Dichtfolie bzw. Dichtplatte 5 beim Einführen in die Öffnung 4 sukzessive vom Rand der Öffnung 4 beginnend von der Öffnung 4 des Reservoirs 3 abhebt. Die Fläche 8 ist dafür so ausgebildet, dass ihr Abstand zur Dichtfolie bzw. Dichtplatte 5 bei Einführen des Öffners 7 in die Öffnung 4 des Reservoirs 3 den kleinsten Abstand zur Dichtfläche der Dichtfolie oder Dichtplatte 5 am Innenrand der Öffnung 4 aufweist. Der Öffner 7 weist außerdem an der Stelle, die beim Einführen des Öffners 7 in die Öffnung 4 den kleinsten Abstand zur Dichtfläche der Dichtfolie bzw. Dichtplatte 5 bildet einen Lochdorn 10 auf, der die Dichtfolie bzw. Dichtplatte 5 beim ersten Kontakt des Öffners 7 mit der Dichtfolie bzw. Dichtplatte 5 durchsticht und somit das Öffnen der Dichtfolie oder Dichtplatte 5 erleichtert. Mit dem Lochdorn 10 kann insbesondere ein willkürliches Einreißen der Dichtfolie oder Dichtplatte 5 verhindert werden. Alternativ oder zusätzlich kann die Fläche 8 am äußeren Rand eine Schneidkante aufweisen, die das Öffnen der Dichtfolie oder Dichtplatte 5 erleichtert, sodass nur ein geringer manueller Druck aufgebracht werden muss, um die Dichtfolie bzw. Dichtplatte 5 zu durchdringen.

Der Öffner 7 ist außerdem in Richtung der Öffnung 4 wenigstens mit einer Länge ausgebildet, dass die Fläche 8 die Dichtfolie bzw. Dichtplatte 5 beim Einführen in die Öffnung 4 nach dem ersten Kontakt des Öffners 7 mit der Dichtfläche bzw. Dichtplatte 5 vollständig durchdringt. Im dargestellten Beispiel der Figur 3 weist das Reservoir 3 eine kreisförmige Öffnung 4 auf. Der Öffner 7 ist komplementär zu dieser Öffnung 4 geformt, d. h. kann passgenau in die Öffnung 4 eingeführt werden. Die Länge des Öffners 7 in Richtung der Öffnung 4 beträgt eine Mindestlänge, die dem Quotienten aus dem Sinus des Winkels, den die Fläche 8 des Öffners 7 und die Dichtfolie bzw. Dichtplatte 5 bei Kontakt der Fläche 8 mit der Dichtfolie bzw. Dichtplatte 5 bildet, und dem Durchmesser des Öffners 7 bzw. der Öffnung 4 entspricht, sodass die Fläche 8 des Öffners 7 die Dichtfolie bzw. Dichtplatte 5 beim Einführen in die Öffnung 4 vom Rand beginnend vollständig durchdringen kann. Die Mindestlänge des Öffners 7 entspricht somit der Mindesteindringtiefe des Öffners 7 in die Öffnung 4 bzw. in das Reservoir 3 nach dem ersten Kontakt des Öffners 7 mit der Dichtfolie bzw. Dichtplatte 5.

Bevorzugt ist der Öffner 7 in Richtung der Öffnung 4 mit einer Länge ausgebildet, mit der der Öffner 7 nachdem die Dichtfolie bzw. Dichtplatte 5 vollständig von der Fläche 8 durchdrungen wurde, noch so weit in die Öffnung 4 bzw. das Reservoir 3 eingeführt werden kann, dass der Öffner 7 die abgetrennte Dichtfolie bzw. Dichtplatte 5 an die Innenwand der Öffnung 4 bzw. des Reservoirs 3 bewegt und dort fixiert. Dies hat den Vorteil, dass der Auslass 9 beim Austreten der Substanz aus dem Reservoir 3 nicht von der losen Dichtfolie bzw. Dichtplatte 5 verschlossen werden kann. Bevorzugt beträgt die Länge des Öffners 7 die genannte Mindestlänge zum vollständigen Durchdringen der Dichtfolie bzw. Dichtplatte 5 zuzüglich mindestens 50%, besonders bevorzugt 75% des Innendurchmessers der Öffnung 4 bzw. bei nichtrunden Öffnungen 4 des längsten Abstandes zwischen den Innenwänden der Öffnung 4 bzw. des Reservoirs 3.

Im gezeigten Beispiel ist der Öffner 7 als Ring mit zwei schmalen Stegen 15 ausgebildet, die den Öffner 7 mit dem Probenaufnehmer 6 verbinden, wobei die Stege 15 zur Länge des Öffners 7 in Richtung der Öffnung 4 beitragen. Durch die Stege 15 werden zwei seitliche Austrittsöffnungen 12 am Öffner 7 gebildet, durch die die Substanz in Richtung des sukzessive gebildeten Auslasses 9 austreten kann. Der Öffner 7 weist außerdem eine Durchführung 11 auf, durch die die Substanz zusätzlich in Richtung des Auslass 9 aus dem Reservoir 3 austreten kann. Der Strömungswiderstand des Öffners 7 ist somit sehr gering und die Substanz kann sowohl aus einer seitlichen Richtung in Bezug auf den Auslass 9 als auch aus einer vertikalen Richtung in Bezug auf den Auslass 9 durch diesen aus dem Reservoir 3 austreten, sodass das Reservoir 3 schnell und vollständig entleert werden kann.

Eine Außenkontur der Stege 15 ist komplementär zur Seitenwand der Öffnung 4 und der Innenwand des Reservoirs 3 geformt. Einer der Stege 15 ist außerdem in einem Bereich angeordnet, in dem die Fläche des Öffners 7 beim ersten Kontakt mit der Dichtfolie oder Dichtplatte 5 einen maximalen Abstand zur Öffnung 3 aufweist. Die Dichtfolie oder Dichtplatte 5 kann dadurch beim Einführen des Öffners 7 in die Öffnung 4 an die Seitenwand der Öffnung 4 und die Innenwand des Reservoirs 3 bewegt bzw. umgeklappt werden und dort von diesem Steg, der in Figur 3 der kürzere der beiden Stege 15 ist, fixiert werden.

Die Probeneinheit 1 der Vorrichtung in Figur 3 ist ferner mit einem Mantel 18 bzw. einer Hülse ausgestaltet, der über weitere schmale Stege 16 mit dem Öffner 7 verbunden ist. Der Mantel 18 umschließt den Öffner 7 und ist in Richtung des Probenaufnehmers 6 und der Fläche 8 des Öffners 7, die in die Öffnung 4 des Reservoirs 3 eingeführt wird, geöffnet. Die Innenfläche des Mantels 18 ist komplementär konturiert zu einer Außenfläche des Substanzbehältnisses 2, die um die Öffnung 4 im Reservoir 3 gebildet ist, ausgebildet, sodass der Mantel 18 das Substanzbehältnis 2 beim Einführen des Öffners 7 in die Öffnung 4 des Reservoirs 3 kraft- bzw. reibschlüssig an dieser Fläche abdichtet. Neben dieser Fläche können am Mantel 18 oder dem Substanzbehältnis 2 noch weitere Dichtflächen 17 vorgesehen sein, an denen das Substanzbehältnis 2 und das Probenbehältnis 1 gegebenenfalls mit zusätzlichem Dichtmaterial gegen die Umgebung abgedichtet werden können. Der Mantel 18 der Probeneinheit 1 schützt einerseits den Öffner 7, insbesondere spitze oder scharfkantige Bereich des Öffners 7, vor ungewollten Berührungen bei der Verwendung und bietet andererseits mit seiner Außenfläche eine Haltefläche für die Handhabung der Probeneinheit 1 beim Aufnehmen einer flüssigen Probe mit dem Probenaufnehmer 6. Darüber hinaus weist der Mantel 18 eine umlaufende Verbindungsstelle 18 für die Befestigung eines Laborgefäßes auf. Der Probenaufnehmer 6 ragt für das Aufnehmen einer flüssigen Probe aus der Probeneinheit 1 hervor und ist im dargestellten Beispiel am Ende, das aus der Probeneinheit 1 hervorragt, mit einer konkav gewölbten Endfläche ausgebildet.

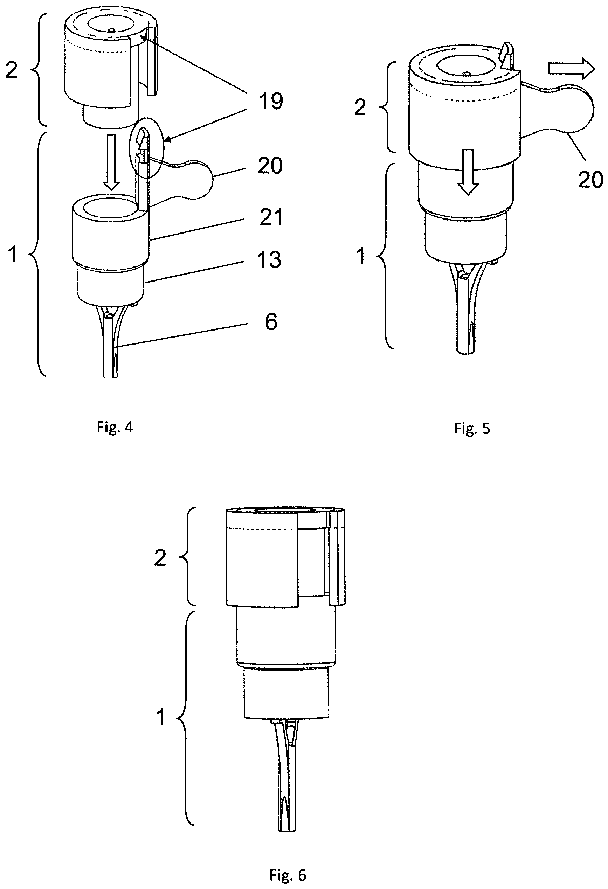

In den Figuren 4, 5 und 6 ist in schematischen, seitlichen Ansichten ein weiteres Beispiel einer Vorrichtung dargestellt. Die Vorrichtung weist unter anderem die Merkmale der Vorrichtung aus Figur 3 auf. Für eine einfache Handhabung der Vorrichtung ist das Substanzbehältnis 2 außerdem an der Probeneinheit 1 befestigbar und das Substanzbehältnis 2 und das Probenbehältnis 1 sind ausgebildet, den Öffner 7 erst mittels eines Drucks auf das Substanzbehältnis 2 in die Öffnung 4 des Reservoirs 3 einzuführen, um die Dichtfolie oder Dichtplatte 5 von der Öffnung 4 abzuheben.

Figur 4 zeigt die Vorrichtung im zerlegten Zustand vor ihrer Verwendung. Die Probeneinheit 1 weist eine Befestigungsvorrichtung 19 und eine Auslösevorrichtung 20 auf. Die Befestigungsvorrichtung 19 ist mittels eines Schnapphakens bzw. Einrastkopfes lösbar am Substanzbehältnis 2 befestigbar. Der Schnapphaken bzw. Einrastkopf ist im gezeigten Beispiel am Mantel 18 Probeneinheit 1 angeordnet, der den Öffner 7 umschließt. Das Substanzbehältnis 2 wird, wie in Figur 4 mit einem Pfeil angedeutet, zum Befestigen so auf die Probeneinheit 1 geschoben, dass der Öffner 7 an der Öffnung 4 des Reservoirs 3 platziert ist und der Schnapphaken an einem Rand des Substanzbehältnisses 2 einrastet bevor der Öffner 7 die Dichtfolie oder Dichtplatte 5 in der Öffnung 4 des Reservoirs 3 berühren kann. Die Probeneinheit 1 kann hierfür beispielsweise an der Haltefläche 21 des Mantels 18 gehalten werden, die von der Verbindungsstelle 13 abgesetzt ist.

In Figur 5 ist die Vorrichtung in einem zusammengefügten Zustand mit geschlossener Befestigungsvorrichtung 19 vor dem Öffnen der Dichtfolie oder Dichtplatte 5 gezeigt. Der Schnapphaken ist in die dafür vorgesehene Nut am Rand des Substanzbehältnisses 2 formschlüssig eingerastet und das Substanzbehältnis 2 an der Probeneinheit 1 befestigt. Der Schnapphaken kann beispielsweise durch einen Zug an der Zuglasche der Auslösevorrichtung 20, wie mit dem Pfeil in Figur 5 angedeutet, aus der Nut am Substanzbehältnis 2 gelöst bzw. entfernt werden, sodass die relative Bewegung des Substanzbehältnisses 2 zur Probeneinheit 1 freigegeben wird und das Substanzbehältnis 2, wie mit dem Pfeil angedeutet, in die Probeneinheit 1 geschoben werden kann, wobei der Öffner 7 die Dichtfolie bzw. Dichtplatte 5 des Reservoirs 3 mit einer einzigen vertikalen Bewegung öffnet. Figur 6 zeigt die Vorrichtung aus Figur 4 und 5 entsprechend mit einer gelösten Schnappbefestigung nach dem Öffnen der Dichtfolie bzw. Dichtplatte 5.

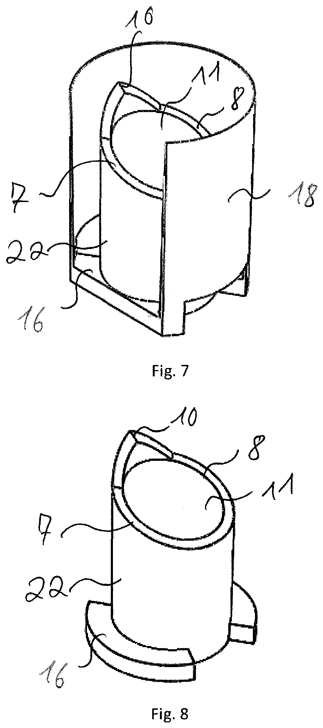

In Figur 7 in einer eine schematischen perspektivischen Ansicht ein Ausführungsbeispiel wiedergegeben, in dem das Grundkonzept des in Figur 3 gezeigten Ausführungsbeispiels verwendet wird, nun jedoch der Öffner 7 nicht als Ring mit vergleichsweise schmalen Durchmesser, beispielsweise gerade einer Höhe des Lochdorns 10 oder weniger, sondern als einseitig abgeschrägter Hohlzylinder ausgebildet ist. Wie bei dem in Figur 3 gezeigten Ausführungsbeispiel ist der Öffner7 von dem Mantel 18 vollständig umschlossen, der in der Darstellung der Figur 7 lediglich zur besseren Verdeutlichung des Aufbaus teilweise nicht eingezeichnet ist. Eine abgeschrägte Fläche des Öffners 7 ist wie bei dem in Figur 3 dargestellten Ausführungsbeispiel ausgestaltet, anstelle der parallel zu dem Mantel 18 verlaufenden Stege 15, die bei der Anwendung typischerweise vertikal angeordnet sind, ist nun jedoch eine Mantelfläche 22 des Hohlzylinders vorgesehen, der einen Innenraum des Öffners 7 vollständig umschließt. Mittig in dem Öffner 7 und von der Mantelfläche 22 umschlossen befindet sich die Öffnung 7.

Die Mantelfläche 22 ist bei dem in Figur 7 dargestellten Ausführungsbeispiel mit dem Mantel 18 über zwei Stege 16 verbunden, die in Draufsicht auf den Lochdorn 10 um 90° bzw. 270° versetzt zu diesem angeordnet sind. Die Mantelfläche 22, die Stege 16 und der Mantel 18 sind hierbei einteilig bzw. einstückig miteinander ausgebildet. Am unteren Ende sind im Bereich des Lochdorns 10 und dem Lochdorn 10 gegenüberliegend zwei Durchbrechungen vorgesehen, d..h ein nicht von einem der beiden Stege 16 überdeckter Zwischenraum. Die Stege 16 sind in diesem Ausführungsbeispiel derart breit, dass von einem der Stege 16 mindestens ein Viertel eines Zwischenraums zwischen der Mantelfläche 22 und dem Mantel 18 bedeckt bzw. überdeckt wird. Die Stege 16 sind im wiedergegebenen Ausführungsbeispiel aus dem gleichen Werkstoff wie der Öffner 7 sowie einteilig bzw. einstückig mit dem Öffner ausgebildet.

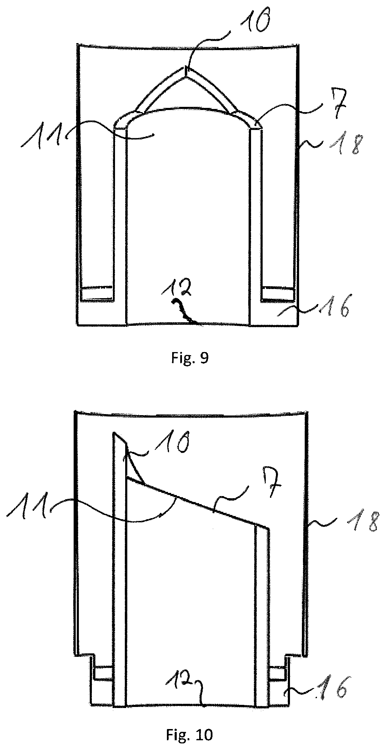

In Figur 8 ist in einer Figur 7 entsprechenden Ansicht der Öffner 7 ohne den Mantel 18 gezeigt. Die Stege 16 sind nicht komplett umlaufend um die Mantelfläche 22 und somit den Öffner 7 ausgebildet, sondern weisen die bereits genannten Aussparungen bzw. Durchbrechungen auf. Figur9 zeigt die in Figur 7 wiedergegebene Anordnung in einer Schnittansicht, aus der deutlich wird, dass ein Hohlraum des Hohlzylinders sich zwischen der Öffnung 11 und der Austrittsöffnung 12 erstreckt. In Figur 10 ist ebenfalls in einer Schnittansicht die Anordnung aus Figur 7 gezeigt, nun verläuft der Schnitt aber mittig durch den Lochdorn 10 entlang der Längsachse der Anordnung, ist also um 90° gegenüber der Schnittansicht der Figur 9 gedreht.

## Patentansprüche

1. Vorrichtung zum Aufnehmen und Handhaben einer flüssigen Probe und einer Substanz, aufweisend eine Probeneinheit (1) und ein Substanzbehältnis (2), bei der
das Substanzbehältnis (2) ein Reservoir (3) aufweist, das ausgebildet ist, eine Substanz im Reservoir (3) aufzubewahren,
das Reservoir (3) eine Öffnung (4) aufweist, die von einer Dichtfolie oder Dichtplatte (5) verschlossen ist, sodass die Substanz im Reservoir (3) gegen die Umgebung abgedichtet ist,
die Probeneinheit (1) einen Probenaufnehmer (6) und einen Öffner (7) aufweist,
der Probenaufnehmer (6) ausgestaltet ist, eine flüssige Probe aufzunehmen und zu halten,
der Öffner (7) ausgebildet ist, zumindest teilweise in die Öffnung (4) des Reservoirs (3) eingeführt zu werden und die Dichtfolie oder Dichtplatte (5) von der Öffnung (4) abzuheben, wobei
eine in Richtung der Dichtfolie oder Dichtplatte angeordnete Fläche (8) des Öffners (7), die in die Öffnung (4) eingeführt wird und die Dichtfolie oder Dichtplatte (5) von der Öffnung (4) abhebt, so ausgebildet ist, dass die Fläche (8) bei Kontakt mit der Dichtfolie oder Dichtplatte (5) in einem schrägen Winkel zur Oberfläche der Dichtfolie oder Dichtplatte (5) ausgerichtet ist und die Dichtfolie oder Dichtplatte (5) beim Einführen in die Öffnung (4) von der Öffnung (4) sukzessive abhebt, sodass ein Auslass (9) in der Dichtfolie oder Dichtplatte (5) gebildet wird, durch den die Substanz aus dem Reservoir (3) austritt und mit der Probe des Probenaufnehmers (2) mischbar ist, **dadurch gekennzeichnet dass**,
der Öffner (7) als Ring ausgebildet ist, der über Stege (15, 16) mit dem Probenaufnehmer (6) verbunden ist.

2. Vorrichtung nach Anspruch 1, **dadurch gekennzeichnet, dass** die Fläche (8) des Öffners (7), die in die Öffnung (4) eingeführt wird und die Dichtfolie oder Dichtplatte (5) von der Öffnung (4) abhebt, ausgebildet ist, die Dichtfolie oder Dichtplatte (5) sukzessive vom Rand der Öffnung (4) beginnend abzuheben.

3. Vorrichtung nach einem der vorhergehenden Ansprüche, **dadurch gekennzeichnet, dass** der Öffner (7) eine Spitze, einen Vorstecher, einen Lochdorn (10) und/oder eine Schneidkante aufweist, der die Dichtfolie oder Dichtplatte (5) bei Kontakt mit der Dichtfolie oder Dichtplatte (5) durchtrennt.

4. Vorrichtung nach einem der vorhergehenden Ansprüche, **dadurch gekennzeichnet, dass** der Öffner (7) komplementär geformt zu der Öffnung (4) des Reservoirs (3) ausgebildet ist.

5. Vorrichtung nach einem der vorhergehenden Ansprüche, **dadurch gekennzeichnet, dass** der Öffner (7) mindestens eine Durchführung (11) und/oder mindestens eine Austrittsöffnung (12) aufweist, durch die die Substanz aus dem Reservoir (3) beim Abheben der Dichtfolie oder Dichtplatte (5) austritt.

6. Vorrichtung nach einem der vorhergehenden Ansprüche, **dadurch gekennzeichnet, dass** der Öffner (7) eine Außenkontur und/oder Außenkante aufweist, die derart geformt ist, dass bei Einführen des Öffners (7) in die Öffnung (4) ein Spalt zwischen der Außenkontur und/oder Außenkante des Öffners (7) und einer Seitenwand der Öffnung (4) und/oder Innenwand des Reservoirs (3) gebildet wird, in dem die Dichtfolie oder Dichtplatte (5) fixiert wird.

7. Vorrichtung nach einem der vorhergehenden Ansprüche, **dadurch gekennzeichnet, dass** das Substanzbehältnis (2) an der Probeneinheit (1) befestigbar ist und ausgebildet ist, den Öffner (7) mittels einer Druck- und/oder Drehbewegung an der Probeneinheit (1) oder dem Substanzbehältnis (2) in die Öffnung (4) einzuführen und die Dichtfolie oder Dichtplatte (5) von der Öffnung (4) abzuheben.

8. Vorrichtung nach einem der vorhergehenden Ansprüche, **dadurch gekennzeichnet, dass** die Probeneinheit (1) eine Verbindungsstelle (13) aufweist, an der ein Behältnis (14) befestigbar ist, das den Probenaufnehmer (6) und die Öffnung (4) des Reservoirs (3) umschließt.

9. Vorrichtung nach einem der vorhergehenden Ansprüche, **dadurch gekennzeichnet, dass** das Substanzbehältnis (2) mehrere Reservoirs (3) und die Probeneinheit (1) mehrere Öffner (7) aufweist.

10. Vorrichtung nach Anspruch 9, **dadurch gekennzeichnet, dass** die Öffner (7) unterschiedliche Längen aufweisen, sodass die Dichtfolien oder Dichtplatten (5) der Reservoirs (3) beim Einführen der Öffner (7) in die Öffnungen (4) der Reservoirs (3) nacheinander geöffnet werden.

11. Verfahren zum Aufnehmen und Handhaben einer flüssigen Probe und einer Substanz mit einer Vorrichtung nach einem der Ansprüche 1 bis 10, bei dem
mit einem Probenaufnehmer (6) einer Probeneinheit (1) eine flüssige Probe aufgenommen wird,
ein Öffner (7) der Probeneinheit (1), der als Ring ausgebildet ist, der über Stege (15, 16) mit dem Probenaufnehmer (6) verbunden ist, in die Öffnung (4) eines Reservoirs (3) eines Substanzbehältnisses (5) eingeführt wird, und
der Öffner (7) eine Dichtfolie oder Dichtplatte (5), mit der die Öffnung (4) des Reservoirs (3) verschlossen ist, von der Öffnung (4) abhebt, sodass eine Auslass (9) gebildet wird, durch den eine Substanz aus dem Reservoir (3) austritt und mit der Probe des Probenaufnehmers (6) mischbar ist.

## Claims

1. A device for taking up and handling a liquid sample and a substance having a sample unit (1) and a substance container (2), in which
the substance container (2) has a reservoir (3) that is configured to store a substance in the reservoir (3);
the reservoir (3) has an opening (4) that is closed by a sealing film or a sealing plate (5) so that the substance is sealed toward the environment in the reservoir (3);
the sample unit (1) has a sampler (6) and an opener (7);
the sampler (6) is configured to take up and hold a liquid sample;
the opener (7) is configured to be at least partially introduced into the opening (4) of the reservoir (3) and to raise the sealing film or sealing plate (5) from the opening (4), wherein
a surface (8) of the opener (7) that is arranged in the direction of the sealing film or sealing plate and that is introduced into the opening (4) and raises the sealing film or sealing plate (5) from the opening (4) is configured such that the surface (8) is aligned at an oblique angle to the surface of the sealing film or sealing plate (5) on contact with the sealing film or sealing plate (5) and raises the sealing film or sealing plate (5) successively from the opening (4) on the introduction into the opening (4) so that an outlet (9) is formed in the sealing film or sealing plate (5) through which the substance exits the reservoir (3) and is miscible with the sample of the sampler (2), **characterized in that**
the opener (7) is formed as a ring that is connected to the sampler (6) via webs (15, 16).

2. A device in accordance with claim 1, **characterized in that** the surface (8) of the opener (7) that is introduced into the opening (4) and that raises the sealing film or sealing plate (5) from the opening (4) is configured to raise the sealing film or sealing plate (5) successively starting from the margin of the opening (4).

3. A device in accordance with one of the preceding claims, **characterized in that** the opener (7) has a tip, a pricker, a piercer (10), and/or a cutting edge that cuts the sealing film or sealing plate (5) on contact with the sealing film or sealing plate (5).

4. A device in accordance with one of the preceding claims, **characterized in that** the opener (7) is formed as complementary with the opening (4) of the reservoir (3).

5. A device in accordance with one of the preceding claims, **characterized in that** the opener (7) has at least one passage (11) and/or at least one outlet opening (12) through which the substance exits the reservoir (3) on the raising of the sealing film or sealing plate (5).

6. A device in accordance with one of the preceding claims, **characterized in that** the opener (7) has an outer contour and/or outer edge that is/are shaped such that, on the introduction of the opener (7) into the opening (4), a gap is formed between the outer contour and/or outer edge of the opener (7) and a side wall of the opening (4) and/or inner wall of the reservoir (3) in which the sealing film or sealing plate (5) is fixed.

7. A device in accordance with one of the preceding claims, **characterized in that** the substance container (2) is fastenable to the sample unit (1) and is configured to introduce the opener (7) into the opening (4) by means of a compressive movement and/or rotational movement at the sample unit (1) or at the substance container (2) and to raise the sealing film or sealing plate (5) from the opening (4).

8. A device in accordance with one of the preceding claims, **characterized in that** the sample unit (1) has a connection point (13) at which a container (14) is fastenable that surrounds the sampler (6) and the opening (4) of the reservoir (3).

9. A device in accordance with one of the preceding claims, **characterized in that** the substance container (2) has a plurality of reservoirs (3) and the sample unit (1) has a plurality of openers (7).

10. A device in accordance with claim 9, **characterized in that** the openers (7) have different lengths so that the sealing films or sealing plates (5) of the reservoirs (3) are opened after one another on the introduction of the openers (7) into the openings (4) of the reservoirs (3).

11. A method of taking up and handling a liquid sample and a substance having a device in accordance with one of the claims 1 to 10, in which
a liquid sample is taken up by a sampler (6) of a sample unit (1);
an opener (7) of the sample unit (1), which opener is formed as a ring that is connected to the sampler (6) via webs (15, 16), is introduced into the opening (4) of a reservoir (3) of a substance container (5); and
the opener (7) raises a sealing film or sealing plate (5) by which the opening (4) of the reservoir (3) is closed from the opening (4) so that an outlet (9) is formed through which a substance exits the reservoir (3) and is miscible with the sample of the sampler (6).

## Revendications

1. Dispositif pour recevoir et manipuler un échantillon liquide et une substance, présentant une unité d'échantillon (1) et un récipient de substance (2), pour lequel
le récipient de substance (2) présente un réservoir (3) qui est réalisé pour conserver une substance dans le réservoir (3),
le réservoir (3) présente une ouverture (4) qui est fermée par une feuille d'étanchéité ou plaque d'étanchéité (5), de sorte que la substance dans le réservoir (3) est rendue étanche par rapport à l'environnement,
l'unité d'échantillon (1) présente un dispositif de réception d'échantillon (6) et un dispositif d'ouverture (7),
le dispositif de réception d'échantillon (6) est conçu pour recevoir et pour retenir un échantillon liquide,
le dispositif d'ouverture (7) est réalisé pour être introduit au moins partiellement dans l'ouverture (4) du réservoir (3) et pour soulever la feuille d'étanchéité ou plaque d'étanchéité (5) de l'ouverture (4), dans lequel
une surface (8) du dispositif d'ouverture (7) disposée en direction de la feuille d'étanchéité ou plaque d'étanchéité, qui est introduite dans l'ouverture (4) et soulève la feuille d'étanchéité ou plaque d'étanchéité (5) de l'ouverture (4), est réalisée de telle sorte que la surface (8) est orientée, lors du contact avec la feuille d'étanchéité ou plaque d'étanchéité (5), selon un angle oblique par rapport à la surface de la feuille d'étanchéité ou plaque d'étanchéité (5) et la feuille d'étanchéité ou plaque d'étanchéité (5) se soulève successivement de l'ouverture (4) lors de l'introduction dans l'ouverture (4), de sorte qu'une sortie (9) est formée dans la feuille d'étanchéité ou plaque d'étanchéité (5), à travers laquelle la substance sort du réservoir (3) et peut être mélangée avec l'échantillon du dispositif de réception d'échantillon (2), **caractérisé en ce que**
le dispositif d'ouverture (7) est réalisé sous la forme d'un anneau qui est relié au dispositif de réception d'échantillon (6) par des éléments jointifs (15, 16).

2. Dispositif selon la revendication 1, **caractérisé en ce que** la surface (8) du dispositif d'ouverture (7), qui est introduite dans l'ouverture (4) et soulève la feuille d'étanchéité ou plaque d'étanchéité (5) de l'ouverture (4), est réalisée pour soulever successivement la feuille d'étanchéité ou plaque d'étanchéité (5) en commençant par le bord de l'ouverture (4).

3. Dispositif selon l'une quelconque des revendications précédentes, **caractérisé en ce que** le dispositif d'ouverture (7) présente une pointe, un perçoir, un mandrin perforé (10) et/ou une arête de coupe qui sectionne la feuille d'étanchéité ou plaque d'étanchéité (5) lors du contact avec la feuille d'étanchéité ou plaque d'étanchéité (5).

4. Dispositif selon l'une quelconque des revendications précédentes, **caractérisé en ce que** le dispositif d'ouverture (7) est réalisé avec une forme complémentaire à l'ouverture (4) du réservoir (3).

5. Dispositif selon l'une quelconque des revendications précédentes, **caractérisé en ce que** le dispositif d'ouverture (7) présente au moins un passage (11) et/ou au moins une ouverture de sortie (12) par lequel(laquelle) la substance sort du réservoir (3) lorsque la feuille d'étanchéité ou plaque d'étanchéité (5) est soulevée.

6. Dispositif selon l'une quelconque des revendications précédentes, **caractérisé en ce que** le dispositif d'ouverture (7) présente un contour extérieur et/ou bord extérieur qui est formé de telle sorte que, lors de l'introduction du dispositif d'ouverture (7) dans l'ouverture (4), une fente est formée entre le contour extérieur et/ou bord extérieur du dispositif d'ouverture (7) et une paroi latérale de l'ouverture (4) et/ou paroi intérieure du réservoir (3), dans laquelle la feuille d'étanchéité ou plaque d'étanchéité (5) est fixée.

7. Dispositif selon l'une quelconque des revendications précédentes, **caractérisé en ce que** le récipient de substance (2) peut être attaché à l'unité d'échantillon (1) et est réalisé pour introduire le dispositif d'ouverture (7) dans l'ouverture (4) au moyen d'un mouvement de pression et/ou de rotation sur l'unité d'échantillon (1) ou le récipient de substance (2) et pour soulever la feuille d'étanchéité ou plaque d'étanchéité (5) de l'ouverture (4).

8. Dispositif selon l'une quelconque des revendications précédentes, **caractérisé en ce que** l'unité d'échantillon (1) présente un point de liaison (13) sur lequel peut être attaché un récipient (14) qui entoure le dispositif de réception d'échantillon (6) et l'ouverture (4) du réservoir (3).

9. Dispositif selon l'une quelconque des revendications précédentes, **caractérisé en ce que** le récipient de substance (2) présente plusieurs réservoirs (3) et l'unité d'échantillon (1) plusieurs dispositifs d'ouverture (7).

10. Dispositif selon la revendication 9, **caractérisé en ce que** les dispositifs d'ouverture (7) présentent des longueurs différentes, de sorte que les feuilles d'étanchéité ou plaques d'étanchéité (5) des réservoirs (3) sont ouvertes les unes après les autres lorsque les dispositifs d'ouverture (7) sont introduits dans les ouvertures (4) des réservoirs (3).

11. Procédé de réception et de manipulation d'un échantillon liquide et d'une substance avec un dispositif selon l'une quelconque des revendications 1 à 10, selon lequel
un échantillon liquide est reçu avec un dispositif de réception d'échantillon (6) d'une unité d'échantillon (1),
un dispositif d'ouverture (7) de l'unité d'échantillon (1), qui est réalisé sous la forme d'un anneau qui est relié au dispositif de réception d'échantillon (6) par des éléments jointifs (15, 16), est introduit dans l'ouverture (4) d'un réservoir (3) d'un récipient de substance (5), et
le dispositif d'ouverture (7) soulève une feuille d'étanchéité ou plaque d'étanchéité (5), avec laquelle l'ouverture (4) du réservoir (3) est fermée, de l'ouverture (4), de sorte qu'une sortie (9) est formée, à travers laquelle une substance sort du réservoir (3) et peut être mélangée avec l'échantillon du dispositif de réception d'échantillon (6).
